Europäisches Patentamt

(19) European Patent Office

Office européen des brevets

(11) Numéro de publication: **0 088 302**
**B1**

(12) # FASCICULE DE BREVET EUROPEEN

(45) Date de publication du fascicule du brevet:
**27.12.85**

(51) Int. Cl.⁴: **A 61 K 39/108**

(21) Numéro de dépôt: **83101842.9**

(22) Date de dépôt: **25.02.83**

(54) Neurotoxine de E.coli détoxifiée, procédé pour la préparer et compositions immunologiques la contenant.

(30) Priorité: **04.03.82 US 354880**

(43) Date de publication de la demande:
**14.09.83 Bulletin 83/37**

(45) Mention de la délivrance du brevet:
**27.12.85 Bulletin 85/52**

(84) Etats contractants désignés:
**AT BE CH DE FR GB IT LI LU NL SE**

(73) Titulaire: **Smith Kline - RIT Société anonyme dite:, rue du Tilleul, 13, B-1320 Genval (Rixensart) (BE)**

(72) Inventeur: **Dobrescu, Lucia, avenue Gabriel-Emile Lebon, 104, B-1160 Bruxelles (BE)**

(74) Mandataire: **Tasset, Gérard, SMITHKLINE - RIT rue de l'Institut, 89, B-1330 Rixensart (BE)**

(56) Documents cités:
**FR - A - 2 227 861**

**CHEMICAL ABSTRACTS, vol. 89, no. 16, 16 octobre 1978, page 391, no. 135726z, Columbus, Ohio, USA, e.H. RELYVELD: "Detoxification of microbial toxins with glutaraldehyde and their use in the preparation of vaccines"**
**BIOLOGICAL ABSTRACTS, vol. 76, 1982, abrègè no. 18490, Philadelphia, Pennsylvania, USA, F. VAN WIJNENDAELE et al.: "Induction of immunity against Escherichia-coli heat stable entero toxin"**

## Description

La présente invention concerne une neurotoxine de E. coli détoxifiée, le procédé pour la préparer et les compositions immunologiques les contenant.

Il est connu qu'une toxine spécifique appelée neurotoxine et produite par des souches pathogènes de Escherichia coli est l'agent étiologique d'une grave maladie des porcelets, appelée maladie de l'oedeme du porcelet.

La maladie de l'oedème du porcelet qu'on appelle également »oedème intestinal« on »entérotoxémie« est une maldie aigüe répandue dans la plupart des pays et responsable de la mort de 5 à 80% des porcelets. La maladie fait son apparition dans les deux semaines qui suivent le sevrage du porcelet et ses symptômes cliniques sont une ataxie, des convulsions, une paralysie partielle ou complète, un oedème de la partie profonde du derme du front et des paupières, avec comme particularité anatomopathologique un oedème de différents organes, principalement de la paroi de l'estomac, du mésentère du colon spiralé et du cerveau.

La neurotoxine est produite par différents sérotypes de E. coli, lesquels sont bien connus.

Dans Res. Vet. Sci. 1: 17—27, 1960, W. J. SOJKA décrit les sérotypes de E. coli les plus communément isolés de cas de maladie de l'oedème du porcelet et dans Zbl. Vet. Med. (B) 18: 622—33, 1971, H. SCHIMMELPFENNIG indique que la neurotoxine est produite par les sérotypes de E. coli qui prédominent dans les isolats de cas de maladie de l'oedème du porcelet, c'est-à-dire les sérotypes 0138, 0139 et 0141.

Lors d'une infection par une telle souche de E. coli, la neurotoxine est libérée et adsorbée dans l'intestin du porcelet, causant une artériopathie générale qui est responsable des symptômes nerveux et de l'oedème.

On a tenté d'obtenir une immunisation active contre la maladie de l'oedème du poulet au moyen de préparations formées soit d'organismes de E. coli entiers soit de lysats de ces organismes (W. J. SOJKA, »E. coli in domestic animals and poultry« édité par le Commonwealth Agricultural Bureau, 1965, p. 124; E. KANKER, Deuts. Tieraerzt. Wochensch. 78: 182—84, 1971 et K. LUTTER, Monatsch. Veterinaermed. 29: 694—99, 1974) mais ou bien ces préparations ne parviennent pas à protéger les porcelets ou bien elles induisent uniquement une immunité spécifique du sérotype correspondant au sérotype de E. coli utilisé dans la préparation.

Le brevet 4 136 181 des Etats-Unis d'Amérique concerne des vaccins efficaces contre la maladie de l'oedème du porcelet; ces vaccins contiennent de la neurotoxine partiellement purifiée additionnée d'un adjuvant et ils sont administrés par voie intramusculaire ou sous-cutanée.

Dans un article intitulé »Studies on the oedema disease producing toxin of Escherichia coli (Advances in Vet. Med. Suppl. to Sbl. Vet. Med. 29: 25—32, 1978), H. SCHIMMELPFENNIG et R. WEBER rapportent que »le traitement des toxines avec le formaldéhyde entraînait une détoxification complète mais les toxines traitées au formol ne présentaient pas une meilleure réponse anticorps« et ces auteurs concluent que »il n'y a pas d'évidence qu'une anatoxine ait été produite«. De plus, les résultats indiquent que, par comparaison avec la neurotoxine naturelle, la toxine traitée au formol a nettement perdu la faculté de stimuler la production d'anticorps neutralisants.

Le brevet 3 983 229 des Etats-Unis d'Amérique concerne un procédé de préparation de vaccins dans lequel un produit toxique est mis en contact avec du glutaraldéhyde dans des conditions opératoires douces et la réaction est arrêtée dès que le stade d'inactivation est atteint.

Il a maintenant été trouvé et ceci constitue l'objet de la présente invention que, lorsqu'on traite la neurotoxine de E. coli avec du glutaraldéhyde dans des conditions opératoires douces — c'est-à-dire par exemple lorsqu'on traite une solution aqueuse de neurotoxine de E. coli titrant sur souris 57 $DE_{50}$/ml (Dose Effective pour 50% des souris traitées) avec une solution aqueuse 0.01 molaire de glutaraldéhyde à température ambiante — et qu'on arrête la réaction avant d'atteindre un niveau d'inactivation de 90% et, de préférence lorsqu'on atteint un niveau d'inactivation compris entre 50 et 90% — et même entre 70 et 90% — de la neurotoxine elle-même — c'est-à-dire après un temps de réaction compris entre 5 minutes et 2 heures —, on obtient un produit appelé ici »neurotoxine détoxifiée« qui est substantiellement non toxique mais encore fortement immunogène et ainsi de valeur pour l'immunisation de jeunes porcelets (c'est-à-dire âgés d'environ une semaine) contre la maladie de l'œdème.

Le glutaraldéhyde est une agent qui est déjà connu pour réagir avec des antigènes, en formant avec eux des produits de réaction polymérisés branchés et la technique de traitement d'inactivation par le glutaraldéhyde est également connue de l'homme de l'art, de même que la méthode pour arrêter la réaction d'inactivation; ces méthodes comprennent par exemple l'ajout d'un agent bloquant la réaction, comme un sel inorganique (par exemple le bisulfite de sodium) ou un acide aminé (de préférence la lysine ou la glycine).

La neurotoxine de E. coli détoxifiée de l'invention présente plus d'innocuité que la neurotoxine purifiée du brevet 4 136 181 des Etats-Unis d'Amérique et cette neurotoxine purifiée peut être utilisée comme produit de départ pour la préparation de neurotoxine détoxifiée suivant la présente invention.

De manière surprenante, les propriétés immunogènes de la neurotoxine détoxifiée de l'invention contrastent fortement avec les produits traités au glutaraldéhyde et présentant un degré d'inactivation supérieur à 90%, lesquels ne sont pas immunogènes.

Pour l'immunisation de jeune porcelets contre la maladie de l'œdème, la neurotoxine détoxifiée est présentée dans une composition pharmaceutique pour administration intramusculaire ou sous-cutanée, de préférence avec un adjuvant et, sous la forme optimale, elle est additionnée d'un antiseptique comme le thiomersal. Comme adjuvants adéquats, on peut citer ceux qui appartiennent ou groupe des hydroxyde et phosphate d'aluminium, par exemple l'Alhydrogel (un gel d'hydroxide d'aluminium fabriqué et vendu par Superfos Export Co., Copenhague, Danemark) et le rapport neurotoxine/adjuvant est, de préférence, calculé pour une adsorption maximale sur l'adjuvant, comme il est indiqué par exemple dans Symp. Series Immunobiol. Standard. 6 : 177—180, 1967).

L'invention est illustrée par les exemples suivants qui n'en limitent pas la protée; par exemple, dans la production de neurotoxine qui est ici décrite dans les exemples, on utilise une souche de E. coli de sérotype 0139 isolée d'un cas d'infection clinique et il est bien évident que n'importe quelle autre souche de E. coli connue pour être productrice de neurotoxine peut également être utilisée dans le même but.

Exemple 1
Préparation de la neurotoxine
(a) Préparation d'ensemencement

Un échantillon d'une souche de E. coli sérotype 0139 isolée d'un cas typique de maladie de l'oedème du porcelet et conservée à l'état lyophilisé est rehydraté à l'aide d'une solution de sérum physiologique et incubée pendant 18 heures à 37°C dans de boîtes de Pétri contenant chacune 20 ml de milieu solide Tryptose-Agar préparé par mélange de 26 g de milieu Tryptose, 30 g d'Agar Difco (milieu Tryptose et Agar Difco sont des produits fabriqués et vendus par Difco Labs.) avec addition d'eau jusqu'à un litre, le mélange étant ensuite porté à 115°C pendant 45 minutes.

Un milieu de culture liquide est alors préparé comme suit: 30 g de peptone Proteose No 3 (un produit fabriqué et vendu par Difco Labs.) 4 g d'extrait de levure et 5 g de dextrose sont dissous dans un litre d'eau à 60°C. Après refroidissement, on ajoute 5 g de NaCl, 5,05 g de NaHPO$_4$ et 1,2 g de KH$_2$PO$_4$. Le milieu dont le pH est 6,9—7,0 est filtré sur filtre EKS Seitz et réparti dans des flacons de culture de 100 ml.

Ces flacons de culture sont inoculés avec les colonies obtenues sur boîtes de Pétri en employant une colonie par fraction de 20 ml de milieu liquide indiqué ci-avant et incubés pendant 6 heures à 37°C sous agitation sur table oscillante (22 à 24 oscillations par minutes).

(b) Production de E. coli et extraction de la neurotoxine

Dans des flacons de production contenant 300 ml du milieu liquide indiqué ci-avant on inocule des aliquotes de 6 ml de milieu de culture contenant E. coli (c'est-à-dire environ 6.10$^9$ bactéries) et les cultures sont incubées pendant un jour sous agitation sur tables oscillantes (22 à 24 oscillations par minute).

Les récoltes de cinq flacons de production (une série) sont rassemblées et chaque série de résultes est centrifugée pendant deux heures à 2000 G. Le sédiment est ensuite mis en suspension dans 150 ml d'eau distillée.

La suspension cellulaire est soniquée pendant 30 minutes dans un sonicateur Branson Europa modèle J22 (Branson Europe N.V., Soest, Pays-Bas) en maintenant le milieu dans un bain de glace fondante.

Après éclatement des cellules, la suspension est centrifugée pendant deux heures à 2000 G à 5°C pour éliminer les débris cellulaires. Le surnageant est passé sur un filtre stérilisant (0,45 u) Millipore (Millipore est une marque de fabrique de Millipore Corporation) pour donner 100 ml de filtrat dont le titre (déterminé par la méthode au point limite de Read et Muench) est 57 DE$_{50}$, sur souris.

Exemple 2

Détoxification de la neurotoxine et préparation du vaccin

Une aliquote de 900 ml de solution de neurotoxine comme obtenue ci-avant est incubée pendant une heure à température ordinaire avec 4,8 ml d'une solution de 25 g de glutaraldéhyde dans 100 ml d'eau distillée apyrogène. Après ce temps de réaction, la réaction est arrêtée par addition d'un millilitre d'une solution de 48 mg de monochlorhydrate de L-lysine dans 10 ml d'eau distillée apyrogène.

La toxine inactivée est additionnée de 446 ml d'une solution stérile à 2% d'hydroxyde d'aluminium (Alhydrogel, Superfos Export Co., Copenhague, Danemark), de 446 ml de tampon Sorensen (Na$_2$HPO$_4$ 1/10 molaire: 22%; KH$_2$PO$_4$ 1/10 molaire: 78%) et 1,8 ml d'une solution de 1 mg de thiomersal dans 10 ml d'eau distillée. Le pH est ajusté à 6,3 à l'aide d'acide chlorhydrique normal. Le mélange est agité pendant 48 heures à température ambiante et dans l'obscurité; il est ensuite centrifugé.

Le sédiment de centrifugation est séparé et dilué avec le surnageant pour obtenir un volume final de 446 ml. Le produit est réparti dans des flacons en verre contenant des doses unitaires (c'est-à-dire 256 DE$_{50}$) de neurotoxine détoxifiée ou des multiples de cette dose.

Pour l'usage vaccinal, le produit est administré par voie intramusculaire ou sous-cutanée, le volume d'une dose unitaire étant 2 ml.

### Exemple 3

#### Relation entre niveau d'inactivation de la toxine et antigénicité

En utilisant la technique de l'exemple 2 mais en utilisant différentes quantités de glutaraldéhyde et en laissant la réaction se dérouler pendant différents temps, on a obtenu des produits présentant différents taux de neurotoxine résiduelle — c'est-à-dire différents taux d'inactivation — et ces produits ont été testés au point de vue de leur protection chez la souris.

Dans cet essai, les taux d'inactivation des différents produits ont été évalués par un test de toxicité (paralysie et mortalité due à la paralysie) sur souris suivant la technique décrite par H. SCHIMMELPFENNIG dans Fortsch. Vet. Med. 13 : 49—50, 1970 et les taux de protection correspondants chez la souris ont été évalués par administration intraveineuse d'une dose unitaire de 0,45 $DE_{50}$ par souris, suivie, 6 jours plus tard, par une administration d'épreuve de 2,8 $DE_{50}$ de neurotoxine par souris et inoculée par voie intraveineuse.

Les résultats sont repris au Tableau I et indiquent que la protection est associée à un niveau d'inactivation de la toxine compris entre environ 50% et environ 90%.

Tableau I

Antigénicité par rapport au niveau d'inactivation de la neurotoxine

| Contact Concentration en glutaraldéhyde (GT) | Durée | Titre en neurotoxine[*] | | Pourcentage d'inactivation de la neurotoxine[*] | Protection chez les souris[**] |
|---|---|---|---|---|---|
| | | Résiduelle en toxine traitée par GT | Contrôle toxine non traitée | | |
| 0.052 M | 5′ | <4 | | 90 | 0 (4/5) |
| | 30′ | <4 | | 90 | 0 (4/5) |
| | 1 h | <4 | | 90 | 0 (4/5) |
| | 2 h | <4 | 39 | 90 | 0 (5/5) |
| 0.026 M | 5′ | <4 | | 90 | 0 (5/5) |
| | 30′ | <4 | | 90 | 0 (5/5) |
| | 1 h | <4 | | 90 | 0 (5/5) |
| | 2 h | <4 | | 90 | 0 (5/5) |
| 0.013 M | 5′ | 22.3 | | 54 | 100 (0/5) |
| | 30′ | 14.1 | | 71 | 100 (0/5) |
| | 1 h | 5.6 | | 88 | 100 (0/5) |
| | 2 h | 5.6 | 48.5 | 88 | 60 (2/5) |
| 0.065 M | 5′ | 44.7 | | 8 | 100 (0/5) |
| | 30′ | 44.7 | | 8 | 100 (0/5) |
| | 1 h | 44.7 | | 8 | 100 (0/3) |
| | 2 h | 24.3 | | 50 | 100 (0/5) |

[*] Exprimé en $DE_{50}$ par administration intraveineuse.
[**] Exprimée en pourcents (nombre d'animaux paralysés/nombre d'animaux testés).

## Exemple 4

### Réponse immunitaire chez les porcelets

Des doses unitaires du vaccin de l'Exemple 2 (256 $DE_{50}$ dans 2 ml) ont été administrées par voie sous-cutanée ou intramusculaire à 3 groupes de 7 porcelets âgés de 7 jours (appelés groupes Ia, Ib et II) et un rappel avec les mêmes doses unitaires a été administré deux semaines plus tard par voie sous-cutanée au groupe Ia est par voie intramusculaire au groupe Ib et, une semaine plus tard encore, par voie intramusculaire au groupe II.

A l'âge de 5 semaines, chaque porcelet a alors reçu, par voie intraveineuse, une inoculation d'épreuve dont le dosage était 2,2 $DE_{50}$ par kg de poids pour les porcelets du groupe Ia et 1,1 $DE_{50}$ par kg de poids pour les porcelets des groupes Ib et II.

Des groupes de contrôle, appelés groupe de contrôle Ia et groupe de contrôle Ib/II de porcelets âgés de 5 semaines ont également reçu par voie intraveineuse une même dose d'épreuve que, respectivement, chacun des groupes Ia, Ib et II.

Tous les porcelets ont été maintenus sous contrôle pour symptômes nerveux (ataxie, convulsions, paralysie) et mortalité pendant une semaine. Des échantillons de sang ont été prélevés avant et après vaccination et la détection d'anticorps anti-neurotoxine a été effectuée par test de séroneutralisation dans les cellules Vero. A cette fin, les échantillons de sérum sont chauffés à 56° C pendant 30 minutes et des dilutions de 2 en 2 en série dans un milieu de conservation pour culture de tissu sont incubées à 37° C pendant une heure en présence d'un même volume de neurotoxine standard à une dilution prédéterminée qui produit un effet cytotoxique dans au moins 50% des cellules Vero ( $\geq$ 50%).

Une double série d'échantillons de 0,2 ml de mélanges toxine-sérum est inoculée sur des tapis monocellulaires pendant 24 heures à 37° C et le point limite de titration de l'antitoxine est exprimé sous forme de l'inverse de la dernière dilution de sérum qui inhibe l'effet cytotoxique de la toxine standard.

Les résultats obtenus pour chaque porcelet et pour chaque groupe sont donnés dans le Tableau II suivant. Aucune réaction défavorable n'a été détectée après vaccination.

Tableau II

Immunogénicité de la neurotoxine détoxifiée vis-à-vis de la maladie de l'œdème du porcelet

| Groupe | Porcelet No | Après inoculation du challenge Symptômes nerveux | Mortalité | Titre en anticorps Anti-neurotoxine*) avant vaccination | après |
|---|---|---|---|---|---|
| Ia | 234 | — | — | 0 | 1 |
| | 235 | — | + | 0 | 0 |
| | 236 | — | — | 0 | 2 |
| | 237 | + | + | 0 | 0 |
| | 238 | — | — | 0 | 2 |
| | 239 | + | + | 0 | 0 |
| | 240 | — | — | 0 | 2 |
| | Groupe entier | 2/7 (28%) | 3/7 (43%) | Seroconversion: 4/7 (57%) | |
| Contrôle Ia | 230 | + | + | 0 | 0 |
| | 231 | + | + | 0 | 0 |
| | 233 | + | + | 0 | 0 |
| | 227 | + | + | 0 | 0 |

Tableau II (Suite)

| Groupe | Porcelet No | Après inoculation du challenge Symptômes nerveux | Mortalité | Titre en anticorps Anti-neurotoxine*) avant vaccination | après |
|---|---|---|---|---|---|
| | 228 | + | + | 0 | 0 |
| | 23 | + | + | 0 | 0 |
| | Groupe entier | 6/6 (100%) | 6/6 (100%) | Séroconversion: 0/6 (0%) | |
| Ib | 266 | — | — | 0 | 2 |
| | 267 | — | — | 0 | 4 |
| | 268 | — | — | 0 | 2 |
| | 269 | — | — | 0 | 1 |
| | 270 | + | — | 0 | 0 |
| | 271 | — | — | 0 | 0 |
| | 272 | — | — | 0 | 0 |
| | Groupe entier | 1/7 (14%) | 0/7 (0%) | Séroconversion: 4/7 (57%) | |
| II | 283 | — | + | 0 | 0 |
| | 284 | — | — | 0 | 8 |
| | 285 | — | — | 0 | 0 |
| | 286 | — | — | 0 | 0 |
| | 287 | — | — | 0 | 0 |
| | 288 | — | — | 0 | 0 |
| | 289 | — | — | 0 | 0 |
| | Groupe entier | 0/7 (0%) | 1/7 (14%) | Séroconversion: 1/7 (14%) | |
| Contrôle Ib/II | 273 | + | — | 0 | 0 |
| | 290 | + | — | 0 | 0 |
| | 51 | + | + | 0 | 0 |
| | 52 | + | — | 0 | 0 |
| | 53 | — | — | 0 | 0 |
| | 54 | + | — | 0 | 0 |
| | 55 | + | — | 0 | 0 |

0 088 302

Tableau II (Suite)

| Groupe | Porcelet No | Après inoculation du challenge Symptômes nerveux | Mortalité | Titre en anticorps Anti-neurotoxine*) avant vaccination | après |
|---|---|---|---|---|---|
| | 56 | + | + | 0 | 0 |
| | 275 | + | — | 0 | 0 |
| | 276 | + | — | 0 | 0 |
| | 277 | + | — | 0 | 0 |
| | 278 | — | — | 0 | 0 |
| | 280 | — | — | 0 | 0 |
| | 281 | + | — | 0 | 0 |
| | Groupe entier | 11/14 (79%) | 2/14 (14%) | Séroconversion: 0/14 (0%) | |

*) Inverse de la dernière dilution de sérum encore active.

Les chiffres du Tableau II indiquent que, chez les très jeunes porcelets, on obtient une excellente réponse immunitaire avec un schéma de vaccination comprenant 2 injections de 256 $DE_{50}$ à un intervalle de 2 semaines.

La protection conférée était de 57% contre une dose léthale pour 100% des animaux de contrôle soumis à l'inoculation d'épreuve et de 82% contre une dose induisant dans deux groupes d'animaux soumis à l'épreuve respectivement une mortalité de 14% et une morbidité de 79%.

Du Tableau II, il appert également (1) que la protection contre la maladie de l'œdème est en relation étroite avec le taux de séroconversion (57%) lequel est, d'une manière significative, différent du taux de conversion des animaux de contrôle et (2) que le schéma de vaccination impliquant deux injections à une semaine d'intervalle est moins efficient.

Exemple 5

Réponse immunitaire obtenue avec la neurotoxine détoxifiée chez des porcelets âgés de 5 à 8 semaines

A 2 groupes de porcelets âgés de 5 à 8 semaines, on a administré par voie sous-cutanée des doses unitaires ou fractions de doses unitaires avec une administration de rappel 3 semaines plus tard et deux autres groupes de porcelets également âgés de 5 à 8 semaines ont été traités de la même manière avec de la neurotoxine adsorbée sur Alhydrogel dans les mêmes conditions.

Les titres en anticorps anti-neurotoxine après vaccination ont été déterminés par séroneutralisation dans des cellules Vero, 14 jours après administration du rappel.

Le schéma de vaccination et les résultats obtenus sont résumés dans le Tableau III qui montre un bon niveau de séroconversion après administration de la neurotoxine détoxifiée.

7

Tableau III

Séroconversion de porcelets vaccinés avec la neurotoxine détoxifiée

| Vaccin | Schéma de vaccination | Nombre d'animaux | Anticorps anti-neurotoxine | |
|--------|----------------------|------------------|------------------------------|---|
| | | | Nombre de séroconvertis | Titre *) postvaccinal en anti-neurotoxine |
| neurotoxine | $2 \times 64$ ED$_{50}$ à 3 semaines d'intervalle | 2 | 1 | 4 : 0 |
| | $2 \times 256$ ED$_{50}$ à 3 semaines d'intervalle | 5 | 4 | 2 : 0 : 8 : 4 : 64 |
| neurotoxine détoxifiée | $2 \times 64$ ED$_{50}$ à 3 semaines d'intervalle | 2 | 1 | 8/16 : 0 |
| | $2 \times 256$ ED$_{50}$ à 3 semaines d'intervalle | 3 | 3 | 4 : 2 : 16 |

*) Inverse de la dernière dilution de sérum encore active.


**Revendications pour les Etats contractants: BE, CH, DE, FR, GB, IT, LI, LU, NL, SE**

1. Neurotoxine de E. coli détoxifiée mais encore immunogène obtenue par réaction de la neurotoxine avec le glutaraldéhyde, caractérisé en ce que son taux d'inactivation est inférieur à 90%.
2. Neurotoxine de E. coli détoxifiée suivant la revendication 1 dans laquelle le taux d'inactivation est compris entre 50 et 90%.
3. Procédé de détoxification de la neurotoxine de E. coli par réaction avec le glutaraldéhyde, caractérisé en ce que la réaction est effectuée à température ambiante pendant une durée comprise entre 5 minutes et une heure à une concentration 0.01 molaire en glutaraldéhyde.
4. Vaccin contre la maladie de l'œdème du porcelet, caractérisé en ce qu'il comprend une dose de neurotoxine de E. coli détoxifiée, suivant l'une quelconque des revendications 1 et 2.
5. Vaccin contre la maladie de l'œdème du porcelet comprenant une dose efficace de neurotoxine de E. coli détoxifiée suivant l'une quelconque des revendications 1 et 2 absorbée sur un adjuvant du groupe des hydroxyde et phosphate d'aluminium.
6. Procédé de préparation d'un vaccin contre la maladie de l'œdème du porcelet contenant de la neurotoxine de E. coli détoxifiée caractérisé en ce que la neurotoxine de E. coli détoxifiée est obtenue par un procédé suivant la revendication 3.


**Revendications pour l'Etat contractant: AT**

1. Procédé de détoxification de la neurotoxine de E. coli par réaction avec le glutaraldéhyde, caractérisé en ce que la réaction est effectuée à température ambiante pendant une durée comprise entre 5 minutes et une heure à une concentration 0.01 molaire en glutaraldéhyde.
2. Procédé et préparation d'une vaccin contre la maladie de l'œdème du porcelet contenant de la neurotoxine de E. coli détoxifiée, caractérisé en ce que la neurotoxine de E. coli détoxifiée est obtenue par un procédé suivant la revendication 1.


**Patentansprüche für die Vertragsstaaten: BE, CH, DE, FR, GB, IT, LI, LU, NL, SE**

1. Entgiftetes jedoch noch immunogenes Neurotoxin aus E. Coli, erhalten durch Reaktion des Neurotoxins mit Glutaraldehyd, dadurch gekennzeichnet, daß sein Inaktivierungsgrad niedriger als 90% ist.
2. Entgiftetes Neurotoxin aus E. Coli gemäß Anspruch 1, bei dem der Inaktivierungsgrad zwischen 50 und 90% liegt.
3. Verfahren zur Entgiftung des Neurotoxins aus E. Coli durch Reaktion mit Glutaraldehyd, dadurch gekennzeichnet, daß die Reaktion bei Raumtemperatur für eine Dauer von 5 Minuten bis eine Stunde bei einer 0,01 molaren Konzentration an Glutaraldehyd durchgeführt wird.

4. Impfstoff gegen die Ödemkrankheit der Ferkel, dadurch gekennzeichnet, daß er eine Dosis entgiftetes Neurotoxin aus E. Coli gemäß einem der Ansprüche 1 oder 2 enthält.

5. Impfstoff gegen die Ödemkrankheit der Ferkel, der eine wirksame Dosis entgiftetes Neurotoxin aus E. Coli gemäß einem der Ansprüche 1 und 2 enthält, das auf einem Adjuvans aus der Gruppe der Hydroxide und Phosphate von Aluminium adsorbiert ist.

6. Verfahren zur Herstellung eines entgiftetes Neurotoxin aus E. Coli enthaltenden Impfstoffes gegen die Ödemkrankheit der Ferkel, dadurch gekennzeichnet, daß das entgiftete Neurotoxin aus E. Coli nach dem Verfahren gemäß Anspruch 3 erhalten wird.

## Patentansprüche für den Vertragsstaat: AT

1. Verfahren zur Entgiftung des Neurotoxins aus E. Coli durch Reaktion mit Glutaraldehyd, dadurch gekennzeichnet, daß die Reaktion bei Raumtemperatur für eine Dauer von 5 Minuten bis eine Stunde bei einer 0,01 molaren Konzentration an Glutaraldehyd durchgeführt wird.

2. Verfahren zur Herstellung eines entgiftetes Neurotoxin aus E. Coli enthaltenden Impfstoffs gegen die Ödemkrankheit der Ferkel, dadurch gekennzeichnet, daß das entgiftete Neurotoxin aus E. Coli nach dem Verfahren gemäß Anspruch 1 erhalten wird.

## Claims for the Contracting States: BE, CH, DE, FR, GB, IT, LI, LU, NL, SE

1. A detoxified but still immunogenic E. coli neurotoxin obtained by reacting neurotoxin with glutaraldehyde wherein its inactivation level is less than 90%.

2. A detoxified E. coli neurotoxin according to claim 1 wherein the inacitvation level is comprised between 50 and 90%.

3. A process for detoxifying the E. coli neurotoxin by reaction with glutaraldehyde wherein the reaction is performed at room temperature for a duration comprised between 5 minutes and one hour at a glutaraldehyde concentration of 0.01 M.

4. A vaccine against oedema disease of piglets which comprises a dose of a detoxified E. coli neurotoxin according to any of claims 1 and 2.

5. A vaccine against oedema disease of piglets comprising an effective dose of a detoxified E. coli neurotoxin according to any of claims 1 and 2 adsorbed on an adjuvant selected from the group comprising aluminum hydroxide and aluminum phosphate.

6. A process for preparing a vaccine against oedema disease of piglets containing a detoxified E. coli neurotoxin wherein detoxified E. coli neurotoxin is obtained by a process according to claim 3.

## Claims for the Contracting State: AT

1. A process for detoxifying the E. coli neurotoxin by reaction with glutaraldehyde wherein the reaction is performed at room temperature for a duration comprised between 5 minutes and one hour at a glutaraldehyde concentration of 0.01 M.

2. A process for preparing a vaccine against oedema disease of piglets containing a detoxified E. coli neurotoxin wherein detoxified E. coli neurotoxin is obtained by a process according to claim 1.